# EUROPEAN PATENT SPECIFICATION

(11) **EP 3 873 237 B1**
(45) Date of publication and mention of the grant of the patent: **20.09.2023**
(21) Application number: 19795023.1
(22) Date of filing: 01.11.2019
(51) Int. Cl.: A23L 33/21, A23L 33/10, A23L 33/00, A61K 31/715, A61P 1/12

(54) **NUTRITIONAL COMPOSITION COMPRISING UREA AND NON-DIGESTIBLE OLIGOSACCHARIDES**
ERNÄHRUNGSZUSAMMENSETZUNG MIT HARNSTOFF UND NICHTVERDAULICHEN OLIGOSACCHARIDEN
COMPOSITION NUTRITIONNELLE COMPRENANT DE L'URÉE ET DES OLIGOSACCHARIDES NON DIGESTIBLES

(30) Priority: 01.11.2018 EP 18203960
(43) Date of publication of application: 08.09.2021
(73) Proprietor: N.V. Nutricia, 2712 HM Zoetermeer (NL)
(72) Inventor: KNOL, Jan, 3584 CT Utrecht (NL); STAHL, Bernd, 3584 CT Utrecht (NL); BONGERS, Roger, 3584 CT Utrecht (NL)
(74) Representative: Nederlandsch Octrooibureau
(86) International application number: PCT/EP2019/079989
(87) International publication number: WO 2020/089463

(56) References cited:
- WO-A1-2011/149345
- WO-A1-2011/149345
- WO-A1-2017/043962
- WO-A1-2017/043962
- DD-A1- 208 542
- DD-A1- 208 542
- GB-A- 993 719
- GB-A- 993 719
- US-A1- 2011 064 707
- US-A1- 2011 064 707
- P. HUHTANEN ET AL: "Evaluation of between-cow variation in milk urea and rumen ammonia nitrogen concentrations and the association with nitrogen utilization and diet digestibility in lactating cows", JOURNAL OF DAIRY SCIENCE., vol. 98, no. 5, 1 May 2015 (2015-05-01), pages 3182-3196, XP055562777, US ISSN: 0022-0302, DOI: 10.3168/jds.2014-8215
- TADASU URASHIMA ET AL: "Recent Advances in Studies on Milk Oligosaccharides of Cows and Other Domestic Farm Animals", BIOSCI. BIOTECH. BIOCHEM., vol. 77, no. 3, 23 March 2013 (2013-03-23) , pages 455-466, XP055496602, ISSN: 0916-8451, DOI: 10.1271/bbb.120810
- GV COPPA ET AL: "Oligosaccharides in human milk during different phases of lactation", ACTA PAEDIATRICA, WILEY-BLACKWELL PUBLISHING LTD, GB, vol. 88, 2 January 2007 (2007-01-02), pages 89-94, XP071693957, ISSN: 0803-5253, DOI: 10.1111/J.1651-2227.1999.TB01307.X
- SHARON M DONOVAN AND BO LONNERDAL: "Non-Protein Nitrogen and True Protein in Infant Formulas", ACTA PAEDIATRICA SCANDINAVICA, WILEY, ALMQVIST OCH WIKSELL, STOCKHOLM, SE, vol. 78, no. 4, 1 January 1989 (1989-01-01), pages 497-504, XP009143767, ISSN: 0001-656X, DOI: 10.1111/J.1651-2227.1989.TB17927.X
- P. Huhtanen ET AL: "Evaluation of between-cow variation in milk urea and rumen ammonia nitrogen concentrations and the association with nitrogen utilization and diet digestibility in lactating cows", JOURNAL OF DAIRY SCIENCE, vol. 98, no. 5, 1 May 2015 (2015-05-01), pages 3182-3196, XP055562777, US ISSN: 0022-0302, DOI: 10.3168/jds.2014-8215
- Tadasu Urashima ET AL: "Recent Advances in Studies on Milk Oligosaccharides of Cows and Other Domestic Farm Animals", Bioscience, Biotechnology, and Biochemistry, vol. 77, no. 3, 23 March 2013 (2013-03-23) , pages 455-466, XP055496602, JP ISSN: 0916-8451, DOI: 10.1271/bbb.120810

## Description

### FIELD OF THE INVENTION

The present invention relates to nutritional compositions for infants and in particular formulae for infants comprising non-digestible oligosaccharides, protein and non-protein nitrogen sources, for improving the intestinal microbiota.

### BACKGROUND OF THE INVENTION

The human gut harbors a complex microbial ecosystem, the intestinal microbiota, which has been recognized as an essential part of our human physiology. In human adults the intestinal microbiota is considered to be a stable ecosystem, hence the microbial colonization process in early-life, which is heavily intertwined with the maturation of the gastrointestinal tract itself, can be considered as fundamental step in healthy development. Early-life nutrition is a major factor that impacts the developing intestinal microbiota community and breastfeeding infants provides for an optimal intestinal microbiota dominated by Bifidobacterium species. As a consequence, breastfeeding has been associated with a multitude of health benefits related to for example infections, inflammation, allergy, eczema, diarrhea, constipation and so on.

In some cases breastfeeding is inadequate or unsuccessful for medical reasons, or because of a choice not to breastfeed. For such situations infant and follow on formulae have been developed. Commercial infant formulae are commonly used today to provide supplemental or the sole source of nutrition early in life. These formulae comprise a range of nutrients to meet the nutritional needs of the growing infant, and typically include fat, carbohydrate, protein, vitamins, minerals, and other nutrients helpful for optimal infant growth and development. Commercial infant formulae are designed to mimic, as closely as possible, the composition of human milk and at least equally important mimic as good as possible the function of human milk.

In order to positively affect the intestinal microbiota, infant formulae have been developed that include probiotics, hence the Bifidobacteria themselves, or prebiotics, in particular non-digestible oligosaccharides that promote the growth of intestinal microbiota, or that include both, such as for example disclosed in WO 2005/110121 and WO 2017/043962.

Proteins and amino acid supplementation to develop a healthy microbiota ecosystem have also been suggested, such as for instance disclosed in EP 1638418.

Infant formulae are often based on cow's milk. Cow's milk comprises more protein and less non-protein nitrogen (NPN), such as urea, compared to human milk.

In addressing this difference GB 993,719 discloses a specific process to produce an infants' milk which includes the addition of urea to be closer to human milk.

DD 208 542 is concerned with providing more breast milk-like bifidogenic nutrition based on cow's milk by diluting standard formula based on cow's milk to decrease the protein content and supplementing this with lactose and urea.

WO 2013/153071 is a more recent document aiming to more closely mimic the growth functionality of breast milk by focusing on keeping the protein content low, but also advocates that NPN level should be low.

WO 2015/105616 discloses pediatric nutritional compositions comprising a protein source comprising an intact milk protein and a partially hydrolyzed milk protein, wherein about 5% to about 25% of total nitrogen content of the composition is non-protein nitrogen. The focus of NPN here is on small peptides and amino acids and not on urea.

### SUMMARY OF THE INVENTION

From a clinical trial it was found that in infants fed human milk the microbiota was both higher in urease genes and in Bifidobacteria compared to the microbiota of formula fed infants and that the level of urease genes in microbiota of human milk fed infants was closely correlated with the level of Bifidobacteria. Upon analysis it was also found that the currently marketed formulae with the non-digestible oligosaccharides GOS/FOS are much lower in urea concentration than human milk. Subsequently, experiments were designed and performed and it was observed that a combination of urea and non-digestible oligosaccharides synergistically stimulated the growth of Bifidobacteria. For urease positive Bifidobacteria, urea, which releases ammonium upon degradation, was a superior source of nitrogen. Furthermore, besides being a nitrogen source, urea was also an important source of COz which is an essential growth factor for Bifidobacteria. In the presence of non-digestible oligosaccharides as a carbon source the released ammonium together with the released CO₂ aided in stimulating the growth not only of urease positive Bifidobacteria, but also will aid the growth of Bifidobacteria that are urease negative by releasing a nitrogen and CO₂ source. Therefore, a nutritional composition with urea and non-digestible oligosaccharides beneficially further improves the microbiota and related health effects in infants or young children.

### DETAILED DESCRIPTION OF THE INVENTION

The invention thus concerns a nutritional composition, which is an infant formula, a follow on formula or a young child formula, comprising digestible carbohydrates, protein, lipid, urea and non-digestible oligosaccharides, wherein the urea is present in an amount of
a. at least 1.13 wt%, preferably 1.13 to 5.6 wt% based on total protein,
b. at least 0.11 wt%, preferably 0.11 to 0.55 wt% based on dry weight of the composition, and/or
c. at least 15 mg, preferably 15 to 75 mg per 100 ml, and non-digestible oligosaccharides are present in an amount of
d. at least 1.5 wt%, preferably 1.5 to 15 wt% based on dry weight of the composition, and/or
e. at least 0.2 g, preferably 0.2 to 2 g per 100 ml.

### Protein levels

The nutritional composition according to the present invention comprises protein. In the context of the present invention, protein is defined as the sum of protein, peptides and free proteinogenic amino acids. Protein according to the present invention can also be referred to as protein equivalent. Proteinogenic amino acids are the 20 different natural amino acids that can be part of eukaryotic protein and peptides. The level of total protein is derived from the commonly known Kjeldahl nitrogen analysis and multiplication by a factor of 6.25. This gives a level of total protein, sometimes also referred to as crude protein level. Hence total protein is the sum of protein equivalent as defined herein plus non-protein nitrogen (NPN). Methods to determine protein and peptides and free amino acids are known in the art. For example a method wherein first protein an peptides are precipitated followed by common protein determination and free amino acids can be determined in the non-precipitated fraction by HPLC with pre-column derivatization with fluorenylmethyl-chloroformate and UV and fluorescence detection is suitable.

The Kjeldahl conversion factor may vary based on the nitrogen source. For purpose of the present invention, the total level of protein is calculated by using a Kjeldahl conversion factor of 6.25.

The total protein in the present nutritional composition preferably provides 6.4 to 15% of the total calories. Preferably the nutritional composition comprises total protein that provides 7.2 to 11% of the total calories. More preferably the present nutritional composition comprises 1.6 to 3.5 g total protein per 100 kcal, more preferably 1.8 to 2.5 g per 100 kcal, even more preferably 1.8 to 2.4 g per 100 kcal and even more preferably 1.8 to 2.1 g per 100 kcal, most preferably from 1.85 to 2.0 g total protein per 100 kcal. Preferably the composition comprises 2.1 g or less total protein per 100 kcal. A low total protein concentration advantageously is closer to human milk as human milk comprises a lower amount of protein based on total calories than cow's milk. Based on dry weight the present nutritional composition preferably comprises 7.8 to 17 wt% total protein, more preferably 8.7 to 12.2 wt%, even more preferably 9.2 to 11.7 wt% total protein based on dry weight. Based on a ready-to-drink liquid product the nutritional composition preferably comprises 1.1 to 2.4 g total protein per 100 ml, more preferably 1.2 to 1.7 g, even more preferably between 1.3 and 1.6 g total protein protein per 100 ml.

The protein equivalent in the present nutritional composition preferably provides 6 to 14.5% of the total calories. Preferably the nutritional composition comprises protein equivalent that provides 6.8 to 10% of the total calories. More preferably the present nutritional composition comprises 1.5 to 3.45 g protein equivalent per 100 kcal, more preferably 1.7 to 2.5 g per 100 kcal, even more preferably 1.8 to 2.4 g per 100 kcal and even more preferably 1.8 to 2.0 g protein equivalent per 100 kcal. Based on dry weight the present nutritional composition preferably comprises 7.3 to 16.5 wt% protein equivalent, more preferably 8.3 to 12.1 wt%, even more preferably 9.0 to 11.6 wt% protein equivalent based on dry weight. Based on a ready-to-drink liquid product the nutritional composition preferably comprises 1.0 to 2.35 g protein equivalent per 100 ml, more preferably 1.1 to 1.65 g, even more preferably between 1.25 and 1.6 g protein equivalent per 100 ml.

When referring to amounts to 100 ml this relates to packed ready-to-drink liquid products, but also to products that are ready to drink after they have been reconstituted with water from a powder or a concentrate according to instructions.

The source of the protein should be selected in such a way that the minimum requirements for essential amino acid content are met and satisfactory growth is ensured. Hence protein sources based on cows' milk proteins such as whey, casein and mixtures thereof and proteins based on soy, potato or pea are preferred. Also protein from the milk of goats or sheep is suitable to provide the minimum requirements for essential amino acid content so that satisfactory growth is ensured. Hence protein sources based on cows' milk proteins, goat milk protein or sheep milk protein are preferred, such as whey, casein and mixtures thereof. In case whey proteins are used, the protein source is preferably based on acid whey or sweet whey, whey protein isolate or mixtures thereof. Preferably the nutritional composition comprises at least 3 wt% casein based on dry weight. Preferably the casein is intact and/or non-hydrolysed. Preferably, the nutritional composition comprises at least 90 wt% cow's milk proteins based on total protein. In one embodiment, preferably, the nutritional composition comprises at least 90 wt% sheep milk proteins based on total protein. In one embodiment, preferably, the nutritional composition comprises at least 90 wt% goat milk proteins based on total protein.

### Urea

Non-protein nitrogen (NPN) according to the invention refers to other components than protein equivalent, and that contain nitrogen such as urea, nucleotides, polyamines, some phospholipids, ammonia, ammonium salts, non-proteinogenic amino acids etc. The most abundant NPN in human milk is urea. In human milk the amount of urea can approximately be 10-13 % of total nitrogen, resulting in a concentration of about 15 to 35 mg urea per 100 ml, with an average around 30-35 mg per 100 ml. It has been demonstrated that urea in breast milk can be incorporated in plasma protein by the infant. Furthermore, the content of urea in cow's milk is lower than in human milk. Methods are known in the art to determine NPN and urea.

The nutritional composition according to the present invention comprises urea. Urea is also known as carbamide, CO(NH₂)₂. Urea is commercially available from various sources in food grade quality, for example from NuGenTec, CA USA, Spectrum chemical, USA, or it can be enriched from cow's milk as disclosed in US 6,506,305. It was found that urea has a stimulating effect on microbiota, in particular in stimulating the growth of Bifidobacteria.

According to the present invention, in one embodiment the nutritional composition comprises at least 1.13 wt% urea based on total protein, preferably 1.13 to 5.6 wt% urea based on total protein, more preferably 1.45 to 3.7 wt%, more preferably 1.5 to 2.6 wt% urea based on total protein. In an alternative embodiment according to the present invention, the nutritional composition comprises at least 0.11 wt% urea based on dry weight of the composition, preferably 0.11 to 0.55 wt% urea, more preferably 0.15 to 0.37 wt% urea based on dry weight of the composition. In yet a further alternative embodiment according to the present invention, the nutritional composition comprises at least 15 mg per 100 ml, more preferably 15 to 75 mg urea per 100 ml. Preferably according to the present invention, the nutritional composition comprises 15 to 75 mg urea per 100 ml. More preferably the amount of urea is 20 to 60 mg per 100 ml, even more preferably 20 to 50 mg per 100 ml, most preferably 20 to 35 mg urea per 100 ml. Preferably the amount of urea-nitrogen is 3.2 to16 % of the total nitrogen. Preferably the amount of urea-nitrogen is 20 to 100 wt%, more preferably 20 to 80 wt% of total non-protein nitrogen.

### Non-digestible oligosaccharides

The nutritional composition according to the present invention comprises non-digestible oligosaccharides. The non-digestible oligosaccharides are present in an amount of at least 1.5 wt% based on dry weight of the composition, more preferably 1.5 to 15 wt% based on dry weight of the composition, more preferably 1.8 to 11 wt% based on dry weight of the composition. Additionally or alternatively, the non-digestible oligosaccharides are present in an amount of at least 0.2 g per 100 ml, preferably 0.2 to 2 g per 100 ml, more preferably 0.25 to 1.5 g per 100 ml.

The term "non-digestible oligosaccharides" as used in the present invention refers to oligosaccharides which are not digested in the intestine by the action of acids or digestive enzymes present in the human upper digestive tract, e.g. small intestine and stomach, but which are preferably fermented by the human intestinal microbiota. For example, sucrose, lactose, maltose and maltodextrins are considered digestible. In a preferred embodiment, the non-digestible oligosaccharides are capable of stimulating the growth of urease positive Bifidobacteria. In a preferred embodiment, the non-digestible oligosaccharides stimulate the growth of urease positive Bifidobacteria. In a preferred embodiment, the non-digestible oligosaccharides are capable of serving as a carbon source for the growth of urease positive Bifidobacteria. In a preferred embodiment, the non-digestible oligosaccharides are capable serve as a carbon source for the growth of urease positive Bifidobacteria. Non-digestible oligosaccharides have a stimulating effect on intestinal microbiota and in particular it was observed that a combination of urea and non-digestible oligosaccharides synergistically stimulated the growth of Bifidobacteria. The presence of the combination of urea and non-digestible oligosaccharides improve the microbiota in making it more similar to the microbiota of breastfed infants. Hence, the presence of the combination of urea and non-digestible oligosaccharides, synergistically and advantageously results in overall microbiota more similar to the microbiota of infants that are predominantly or exclusively breastfed.

Preferably the present non-digestible oligosaccharides are soluble. The term "soluble" as used herein, when having reference to a polysaccharide, fibre or oligosaccharide, means that the substance is at least soluble according to the method described by L. Prosky et al., J. Assoc. Off. Anal. Chem. 71, 1017-1023 (1988).

Preferably the present nutritional composition comprises non-digestible oligosaccharides with a degree of polymerization (DP) in the range of 2 to 250, more preferably 2 to 60. The non-digestible oligosaccharide is preferably selected from the group consisting of fructo-oligosaccharides, galacto-oligosaccharides, arabino-oligosaccharides, arabinogalacto-oligosaccharides, gluco-oligosaccharides, chito-oligosaccharides, glucomanno-oligosaccharides, galactomannooligosaccharides, mannan-oligosaccharides, fuco-oligosaccharides, sialyl-oligosaccharides and N acetylglucosamine oligosaccharides. The group of fructo-oligosaccharides includes inulins and the group of galacto-oligosaccharides includes transgalacto-oligosaccharides or beta-galacto-oligosaccharides. The group of fuco-oligosaccharides include 2'-fucosyllactose (2'FL), 3-fucosyllactose (3FL) and 3,2'-difucosyllactose (lactodifucotetraose; LDFT). The group of sialyl-oligosaccharides include 3-sialyllactose (3'SL) and 6-sialyllactose (6'SL). The group of N-acetylglucosamine oligosaccharides include lacto-N-tetraose (LNT) and lacto-N-neotetraose (LNnT).

Such non-digestible oligosaccharides share many biochemical properties and have similar functional benefits including improving the microbiota. Yet it is understood that some non-digestible oligosaccharides and preferably some mixtures have an even further improved effect. Therefore more preferably the non-digestible oligosaccharides are selected from the group consisting of fructo-oligosaccharides and galacto-oligosaccharides, and mixtures thereof. More preferably the non-digestible oligosaccharides are selected from the group consisting of fructo-oligosaccharides, galacto-oligosaccharides and mixtures thereof. Even more preferably the non-digestible oligosaccharides are selected from inulin and/or beta-galacto-oligosaccharide, most preferably the non-digestible oligosaccharides comprise beta-galacto-oligosaccharide.

Preferably the non-digestible oligosaccharides comprise galacto-oligosaccharide with beta(1,4), beta(1,3) and/or beta(1,6) glycosidic bonds and preferably such galacto-oligosaccharide with beta(1,4), beta(1,3) and/or beta(1,6) glycosidic bonds comprise a terminal glucose. Transgalactooligosaccharide is for example available under the trade name Vivinal^{®}GOS (Domo FrieslandCampina Ingredients), Bi2muno (Clasado), Cup-oligo (Nissin Sugar) and Oligomate55 (Yakult). These non-digestible oligosaccharides improve the microbiota, in particular Bifidobacteria, to a larger extent.

Preferably the present nutritional composition comprises a mixture of galacto-oligosaccharide and fructo-oligosaccharide. A fructo-oligosaccharide may in other context have names like fructopolysaccharide, oligofructose, polyfructose, polyfructan, inulin, levan and fructan and may refer to oligosaccharides comprising beta-linked fructose units, which are preferably linked by beta(2,1) and/or beta(2,6) glycosidic linkages, and a preferable DP between 2 and 200. Preferably, the fructo-oligosaccharide contains a terminal beta(2,1) glycosidic linked glucose. Preferably, the fructo-oligosaccharide contains at least 7 beta-linked fructose units. In a further preferred embodiment the present nutritional composition comprises a mixture of galacto-oligosaccharide and inulin. Inulin is a type of fructo-oligosaccharide wherein at least 75% of the glycosidic linkages are beta(2,1) linkages. Typically, inulin has an average chain length between 8 and 60 monosaccharide units. A suitable fructo-oligosaccharide for use in the nutritional compositions of the present invention is commercially available under the trade name Raftiline^{®}HP (Orafti). Other suitable sources are Raftilose (Orafti), Fibrulose and Fibruline (Cosucra) and Frutafit and Frutalose (Sensus). Preferably the mixture of galacto-oligosaccharide and fructo-oligosaccharide is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when galacto-oligosaccharide has a low average DP and fructo-oligosaccharide has a relatively high DP. Most preferred is a mixture of galacto-oligosaccharide with an average DP below 10, preferably below 6 and a fructo-oligosaccharide with an average DP above 7, preferably above 11, even more preferably above 20. Such a mixture synergistically improves the intestinal microbiota in infants by making it more similar to the microbiota of breastfed infants.

Preferably the present nutritional composition comprises a mixture of short chain fructo-oligosaccharide and short chain galacto-oligosaccharides. Preferably the mixture of short chain fructo-oligosaccharide and short chain galacto-oligosaccharides is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharide and galacto-oligosaccharides with an average DP below 10, preferably below 6.

Preferably the present nutritional composition comprises a mixture of short chain fructo-oligosaccharide and long chain fructo-oligosaccharide. Preferably the mixture of short chain fructo-oligosaccharide and long chain fructo-oligosaccharide is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharide with an average DP below 10, preferably below 6 and a fructo-oligosaccharide with an average DP above 7, preferably above 11, even more preferably above 20.

In one embodiment, preferably the present nutritional composition comprises 2'-fucosyllactose. Preferably the present nutritional composition comprises a mixture of 2'-fucosyllactose and galacto-oligosaccharides. Preferably 2'-fucosyllactose and galacto-oligosaccharide are present in a weight ratio from 1/99 to 99/1, more preferably from 1/19 to 19/1, even more preferably from 1/10 to 10/1. The presence of 2'-fucosyllactose further improves the microbiota, and when present in a mixture with galacto-oligosaccharide a further improved effect on the microbiota composition and activity is observed.

The non-digestible oligosaccharides have an improved effect on stimulating microbiota, in particular on selectively stimulating the growth of Bifidobacteria, and preferably the non-digestible oligosaccharides are selected from one or more of the group consisting of beta-galacto-oligosaccharides and fructo-oligosaccharides, which have the most pronounced improved effect on stimulating microbiota, in particular on stimulating the growth of Bifidobacteria and especially in combination with urea, these non-digestible oligosaccharides have a synergistic effect on stimulating microbiota, in particular on stimulating the growth of Bifidobacteria.

The non-digestible oligosaccharides have an improved effect on stimulating microbiota, in particular on selectively stimulating the growth of Bifidobacteria, and preferably the non-digestible oligosaccharides are selected from one or more of the group consisting of beta-galacto-oligosaccharides, fructo-oligosaccharides and 2'-fucosyllactose, or mixtures thereof, which have the most pronounced improved effect on stimulating microbiota, in particular on stimulating the growth of Bifidobacteria and especially in combination with urea, these non-digestible oligosaccharides have a synergistic effect on stimulating microbiota, in particular on stimulating the growth of Bifidobacteria.

### Digestible carbohydrates

The nutritional composition according to the invention comprises digestible carbohydrates. The digestible carbohydrates preferably provide 30 to 80% of the total calories of the nutritional composition. Preferably the digestible carbohydrates provide 40 to 60% of the total calories. Based on calories the nutritional composition preferably comprises of 5 to 20 g of digestible carbohydrates per 100 kcal, more preferably 7.5 to 15 g. When in liquid form, e.g. as a ready-to-feed liquid, the nutritional composition preferably comprises 3.0 to 30 g digestible carbohydrate per 100 ml, more preferably 6.0 to 20, even more preferably 7.0 to 10.0 g per 100 ml. Based on dry weight the nutritional composition preferably comprises 20 to 80 wt%, more preferably 40 to 65 wt% digestible carbohydrates.

Preferred digestible carbohydrate sources are lactose, glucose, sucrose, fructose, galactose, maltose, starch and maltodextrin. Lactose is the main digestible carbohydrate present in human milk. Lactose advantageously has a low glycemic index. The nutritional composition preferably comprises lactose. The nutritional composition preferably comprises digestible carbohydrate, wherein at least 35 wt%, more preferably at least 50 wt%, more preferably at least 75 wt%, even more preferably at least 90 wt%, most preferably at least 95 wt% of the digestible carbohydrate is lactose. Based on dry weight the nutritional composition preferably comprises at least 25 wt% lactose, preferably at least 40 wt%.

### Lipid

The nutritional composition according to the present invention comprises lipid. The lipid of the present nutritional composition preferably provides 3 to 7 g per 100 kcal of the nutritional composition, preferably the lipid provides 4 to 6 g per 100 kcal. When in liquid form, e.g. as a ready-to-feed liquid, the nutritional composition preferably comprises 2.1 to 6.5 g lipid per 100 ml, more preferably 3.0 to 4.0 g per 100 ml. Based on dry weight the present nutritional composition preferably comprises 12.5 to 40 wt% lipid, more preferably 19 to 30 wt%. Preferably the lipid comprises the essential fatty acids alpha-linolenic acid (ALA) and linoleic acid (LA). The lipid may be provided as free fatty acids, in triglyceride form, in diglyceride form, in monoglyceride form, in phospholipid form, or as a mixture of one of more of the above. Preferably the present nutritional composition contains at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil and milk fat.

### LC-PUFA

The nutritional composition according to the present invention preferably comprises long chain polyunsaturated fatty acids (LC-PUFA). LC-PUFA are fatty acids wherein the acyl chain has a length of 20 to 24 carbon atoms (preferably 20 or 22 carbon atoms) and wherein the acyl chain comprises at least two unsaturated bonds between said carbon atoms in the acyl chain. More preferably the present composition comprises at least one LC-PUFA selected from the group consisting of eicosapentaenoic acid (EPA, 20:5 n3), docosahexaenoic acid (DHA, 22:6 n3), arachidonic acid (ARA, 20:4 n6) and docosapentaenoic acid (DPA, 22:5 n3), preferably DHA, EPA and/or ARA. Such LC-PUFAs have a further beneficial effect on improving intestinal health.

The preferred content of LC-PUFA in the present nutritional composition does not exceed 15 wt% of total fatty acids, preferably does not exceed 10 wt%, even more preferably does not exceed 5 wt%. Preferably the present composition comprises at least 0.2 wt%, preferably at least 0.25 wt%, more preferably at least 0.35 wt%, even more preferably at least 0.5 wt% LC-PUFA of total fatty acids, more preferably DHA. The present composition preferably comprises ARA and DHA, wherein the weight ratio ARA/DHA preferably is above 0.25, preferably above 0.5, more preferably 0.75-2, even more preferably 0.75-1.25. The weight ratio is preferably below 20, more preferably between 0.5 and 5, even more preferably below 2. The amount of DHA is preferably above 0.2 wt%, more preferably above 0.3 wt%, more preferably at least 0.35 wt%, even more preferably 0.35 - 0.6 wt% on total fatty acids.

### Probiotics

In a preferred embodiment, the present nutritional composition comprises lactic acid producing bacteria. Lactic acid producing bacteria are often probiotic bacteria. The term probiotic is known in the art and refers to micro-organisms, preferably bacteria, which have a beneficial effect on a host when ingested by or administered to that host. Preferably, the present nutritional composition comprises lactic acid producing bacteria of the genus Lactobacillus or Bifidobacterium or of both, more preferably a Bifidobacterium. In a preferred embodiment, the Lactobacillus is one or more selected from *L. rhamnosus, L. casei, L. paracasei, L. helveticus, L. delbrueckii, L. reuteri, L. brevis, L. crispatus, L. sakei, L. jensenii, L. sanfransiscensis, L. fructivorans, L. kefiri, L. curvatus, L. paraplantarum, L. kefirgranum, L. parakefir, L. fermentum, L. plantarum, L. acidophilus, L. johnsonii, L. gasseri, L. xylosus, L. salivarius* etc. Preferred species are *L. rhamnosus, L. casei, L. paracasei, L. reuteri, L. crispatus, .L fermentum L. plantarum L. acidophilus, L. johnsonii L. gasseri and L. salivarius, more preferably one or more selected from L. plantarum, L. casei* and *L. rhamnosus.,* more preferably the probiotic comprises a strain belonging to the species *L. casei.* In a preferred embodiment, the Bifidobacterium is one or more selected from B. *longum, B. breve, B. animalis, B. longum spp infantis, B. bifidum, B. adolescentis, B. pseudolongum, B. catenulatum, B. pseudocatenulatum, B. angulatum.*

In one embodiment, the present nutritional composition with urea and non-digestible oligosaccharides preferably comprises a urease positive Bifidobacterium, more preferably the present nutritional composition comprises B. *longum spp infantis.*

In one embodiment, the present nutritional composition preferably comprises a urease negative Bifidobacterium, more preferably the present nutritional composition comprises *B*. *breve.* As the combination of urea and non-digestible oligosaccharides will directly stimulate the growth of urease positive Bifidobacteria, it is advantageous to add urease negative bacteria. This will beneficially increase the Bifidobacterial biodiversity.

In one embodiment, the present nutritional composition comprises both a urease positive and a urease negative Bifidobacterium, preferably *B*. *breve* as urease negative bacterium as this species is highly prevalent in the microbiota of human milk fed infants, and *B*. *longum spp infantis* as urease positive Bifidobacterium, as this species is also present in the microbiota of human milk fed infants and highly versatile in the use of non-digestible carbohydrates as carbon and energy source. 8. *breve* and *B*. *longum spp infantis* strains are commercially available for example *B*. *breve* Bb-03 (Rhodia/Danisco), *B. breve* M-16V (Morinaga), *B. breve* R0070 (Institute Rosell, Lallemand), B. *breve* BR03 (Probiotical), *B. breve* BR92) (Cell Biotech), DSM 20091, LMG 11613, YIT4065, FERM BP-6223 and CNCM 1-2219. B. *longum spp infantis* strains are commercially available, for example as Bi-26 ( DuPont), M-63 (Morinaga), R-0033 (Lallemand). Alternatively *B*. *breve* or *longum spp infantis* strains can be obtained from culture collections such as ATCC or DSM or can be isolated from the faeces of healthy breast fed infants. The urease positive Bifidobacteria will release CO₂ and nitrogen from the urea and will thus support the growth of urease negative Bifidobacteria. The present nutritional composition preferably comprises 10² to 10¹³ colony forming units (cfu) lactic acid producing bacteria, preferably Bifidobacteria, per gram dry weight of the nutritional composition, preferably 10⁴ to 10¹², more preferably 10⁵ to 10¹⁰, most preferably from 10⁵ to 1×10⁸ cfu lactic acid producing bacteria per gram dry weight of the nutritional composition. Preferably the present nutritional composition comprises 10³ to 10¹³ cfu lactic acid producing bacteria, preferably Bifidobacteria, per 100 ml, more preferably 10⁶ to 10¹¹ cfu per 100 ml, most preferably 10⁷ to 10⁹ cfu lactic acid producing bacteria per 100 ml.

### Formula

The nutritional composition according to the present invention is intended for administration to infants and young children. The present nutritional composition is not human milk. The present nutritional composition is also not native cow's milk or native milk from another mammal The terms "infant formula" or "follow on formula" or "young child formula" mean that it concerns a composition that is artificially made or in other words that it is synthetic. Hence in one embodiment the nutritional composition that is administered is an artificial infant formula or an artificial follow on formula or an artificial young child formula or an artificial growing up milk or a synthetic infant formula or a synthetic follow on formula or a synthetic young child formula or a synthetic growing up milk.

In the present context, infant formula refers to nutritional compositions, artificially made, intended for infants of 0 to about 4 to 6 months of age and are intended as a substitute for human milk. Typically infant formulae are suitable to be used as sole source of nutrition. Such formulae are also known as starter formula. Formula for infants starting at 4 to 6 months of life to 12 months of life are intended to be supplementary feedings for infants that start weaning on other foods. Such formulae are also known as follow on formulae. Infant formulae and follow on formulae are subject to strict regulations, for example for the EU Commission Directive 2006/141/EC. In the present context, young child formula or growing up milk refers to nutritional compositions, artificially made, intended for infants of 12 months to 36 months, which are intended to be supplementary feedings to young children.

The nutritional composition according to the present invention is an infant formula or a follow on formula or a young child formula, more preferably the nutritional composition is an infant formula or a follow on formula, even more preferably an infant formula.

The nutritional composition is preferably an infant formula or follow on formula and preferably comprises 3 to 7 g lipid per 100 kcal, preferably 4 to 6 g lipid per 100 kcal, more preferably 4.5 to 5.5 g lipid per 100 kcal, preferably comprises 1.6 to 3.5 g total protein per 100 kcal, preferably 1.8 to 2.4 g protein per 100 kcal, more preferably 1.85 to 2.0 g protein per 100 kcal, more preferably 1.8 to 2.0 g protein per 100 kcal and preferably comprises 5 to 20 g digestible carbohydrate per 100 kcal, preferably 7.5 to 15 g digestible carbohydrate per 100 kcal,

Preferably the nutritional composition is an infant formula or follow on formula, when ready to drink has an energy density of 60 kcal to 75 kcal per 100 ml, more preferably 60 to 70 kcal per 100 ml. This density ensures an optimal balance between hydration and caloric intake.

In one embodiment, the nutritional composition is a powder. Suitably, the nutritional composition is in a powdered form, which can be reconstituted with water or other food grade aqueous liquid, to form a ready-to drink liquid, or is in a liquid concentrate form that should be diluted with water to a ready-to-drink liquid.

### Application

According to the present invention, upon feeding formulae comprising urea as well as non-digestible oligosaccharides, a stimulating and/or synergistic effect on the growth of urease positive Bifidobacteria is observed, and, by releasing a nitrogen and CO₂ source, also subsequently an impact on the growth of Bifidobacteria that are urease negative is anticipated. Therefore, a nutritional composition with urea and non-digestible oligosaccharides beneficially further improves the microbiota and related health effects in infants or young children. In particular the combination of urea and non-digestible oligosaccharides was effective in achieving an increase in the total content of Bifidobacteria or the level of Bifidobacteria. Additionally the combination of urea and non-digestible oligosaccharides can be effective in achieving an increase of the diversity of Bifidobacteria. In essence, a nutritional composition with urea and non-digestible oligosaccharides beneficially further improves the microbiota and beneficially affects and improves related health effects in infants or young children.

According to the invention, the present nutritional composition is for use in preventing or treating dysbiosis of the intestinal microbiota in infants or young children, preferably infants.

According to the invention, the present nutritional composition is for use in preventing and/or treating disorders associated with a compromised intestinal microbiota in infants or young children, preferably infants. Furthermore, the present invention provides a composition for use in preventing and/or treating a disorder selected from the group consisting of diarrhea, constipation, intestinal inflammation, intestinal infection and allergy in infants or young children, preferably infants.

### EXAMPLES

### Example 1: In breast fed infants the faecal urease is higher than in formula fed infants, and this is correlated with the amount of faecal Bifidobacteria.

A double-blind controlled and randomised study that investigates the effect of partial hydrolysed formula feeding (infant formula and follow on formula, depending on the age of the infants) supplemented with a mixture of non-digestible oligosaccharides in healthy term born infants during their first year of life was performed and compared with a control formula. In this trial a breast-fed reference group was also present. During this study faecal samples of 1 month old infants were taken and analyzed.

The amount of urease was determined using qPCR with degenerate *B. infantis* UreC primers and Bifidobacterium 16S primers. 64 samples were collected of which 25 were from exclusively breast-fed infants and 13 from exclusively formula fed infants. The remainder was of partially breast-fed infants (15 were predominantly breast fed and 11 were predominantly formula fed).

There was a significantly higher abundance of urease genes in the faecal DNA of exclusively breast fed infants (p=0.0285) compared to the exclusively formula fed infants, using Student's T test.

Using the Bifidobacterium 16S primer it could be deduced that there was a statistically significantly higher abundance of Bifidobacterium in the faeces of the exclusively breast-fed infants compared to exclusively formula fed infants using Student's Test (p=0.0008).

The ratio of urease genes based on total of Bifidobacterium was not different among these two groups (p=0.9, Student's T test). This is indicative of a correlation between the urease genes and the amount of Bifidobacteria. This is indicative of a role urea plays in increasing the level of Bifidobacteria.

### Example 2: Currently marketed infant formulae with GOS/lcFOS have low amount of urea.

Infant formula manufacturers use casein and whey protein sources that have been prepared by electrodialysis, ultrafiltration or ion-exchange chromatography, rather than whole cow's milk when preparing formulae. Therefore, the nitrogen composition of the protein sources used for manufacturing (modified) cow's milk based formulae can be quite different and the proportion of NPN, in particular urea, will also vary considerably and can expected to be lower than in natural cow's milk, let alone than in human milk.

Commercially available infant formulae were analysed for their urea content using MUVA-MET 128 Boehringer/R-Biopharm Kit 10 542946035 by muva Kempten, Germany. Protein levels were taken from the pack and are determined by Kjeldahl (conversion factor 6.25). The AR1 formula did not comprise GOS/lcFOS. The other formulae contained scGOS/IcFOS in a 9:1 weight ratio, and in a level of 0.8 g/100 ml.

Results are shown in Table 1.

**Table 1: levels of protein and urea in commercially available products.**

| Formula | Nutrilon Standard 1 | Nutrilon Standard 2 | Nutrilon AR1 | Nutrilon Omneo Comfort 1 |
|---|---|---|---|---|
| gram dry formula needed per 100 ml | 13.6 | 14.6 | 13.2 | 13.7 |
| Kcal/100 ml | 66 | 68 | 66 | 66 |
| Protein sources | Whey, skimmed milk, whey concentrate (W/C 6/4) | Whey, skimmed milk (W/C 1/1) | Skimmed milk (W/C 8/2) | Partially hydrolysed whey protein |
| Total protein g/100 ml | 1.3 | 1.4 | 1.6 | 1.5 |
| Urea mg/100 ml | 10.2 | 9.7 | 8.2 | 4.9 |
| Urea wt%/dry weight | 0.075 | 0.047 | 0.039 | 0.024 |
| Urea wt%/total protein | 0.78 | 0.69 | 0.51 | 0.33 |
| Urea wt%/total N | 2.3 | 2.0 | 1.5 | 0.96 |

As can be concluded from Table 1, the levels of urea in the formula are low and below the levels found typically in human milk. This is the case for a wide range of formulae based on cow's milk protein, irrespective of the protein source, and irrespective if the ratio whey protein/casein (W/C).

### Example 3: Non-digestible oligosaccharides together with urea are superior in stimulating growth of Bifidobacteria

The following strains were used: *Bifidobacterium breve* DSM20213, which is the type strain of *B*. *breve* and urease negative. A mix of *Bifidobacterium longum* subspecies *infantis* strains was used that were predicted to be urease positive, to increase the chance of having a urease activity. For supporting the invention, it does not matter which specific strains are used, as long as they have urease activity. Strains used were a mix of *Bifidobacterium longum* spp *infantis* DSM20088 (type strain), *B. infantis* M-63 (Morinaga), *B. longum* subspecies *infantis* ATCC 17930, *B*. *longum* subspecies *infantis* DN_163_0041 isolated from the faeces of a bottle-fed infants, *B. infantis* DN_163_0046 and *B. longum* ssp. *infantis* R-0033 (Lallemand) and *Bidfidobaccterium bifidum* ATCC29521 (type strain). It was indeed confirmed that the three publicly available single strains alone (*B*. *longum spp infantis* M-63, *B*. *longum subspecies infantis* ATCC 17930, and *B*. *longum ssp. infantis* R-0033) were able to grow very well in the presence of urea and a carbon source and in the absence of CO₂ and other nitrogen source and these single strains can thus also be used instead of the mix (data not shown).

To demonstrate utilization of urea as carbon- and nitrogen source a TOS-Propionate Agar-based fermentation broth with the following final composition was used:
1 g/l yeast extract, 0.75 g/l tryptone, 3 g/l KH₂PO₄, 4.8 g/l K₂HPO₄, 0.2 g/l MgSO₄, 0.5 g Cysteine.HCl, 15 g Sodium Propionate, supplemented with 10 ml vitamin mix and 10 ml metal mix from 100x concentrates (Teusink et al. 2005, AEM, Vol 71 (issue 11) p. 7253-7262) and 100 µM NiCI. The pH of this medium is 6.8 (+/- 0.05). As carbon source 20 g/l lactose was used and when appropriate urea was added to a final concentration of 100 mM. The medium was sterilized using filtration step with a 0.22 µm filter. This medium contained 25% of the amount of tryptone as present in TOS-Priopionate medium which has been shown to be nitrogen limiting. Precultures of bifidobacterium strains were grown statically in absence of urea at 37 °C in an anaerobic atmosphere (90% N₂, 5% H₂, 5% CO₂).

Fermenters were sterilized containing 80 ml demi water and 20 ml of a 20 wt% solution of scGOS/lcFOS in a 9:1 wt/wt ratio. As a source of scGOS VivinalGOS (DOMO, Friesland Campina) was used, and as a source of IcFOS, RaftilinHP was used (Orafti). After sterilization 100 ml of sterilized 2x concentrated BASE medium was added. The final concentration of scGOS/lcFOS was 2 wt% based on volume, and this served as the carbon source. As a nitrogen source tryptone was added (3.0 g/l), or 0, 10, 50 or 100 mM urea (final concentration), corresponding to 0, 0.6 g/l, 3 g/l and 6 g/l. Precultures were grown in static tubes in 1x BASE supplemented with 3.0 g/l tryptone and 2 wt% scGOS/lcFOS under an anaerobic gas atmosphere (90% N₂, 5% H₂, 5% CO₂). Inoculation occurred from a single colonies grown on TOS-mupirocin agar plates as known in the art. The main cultures (in the fermenters) were performed with 1xBASE supplemented with 0, 15, 50 or 100 mM urea (if applicable). During fermentation the headspace was flushed with N₂ gas or with an anaerobic gas mixture ((90% N₂, 5% H₂, 5% CO₂). CO₂ is known to be needed for the growth of most Bifidobacteria in the absence of substrates that can liberate CO₂.

The main fermenters were inoculated with the preculture of *B*. *breve* at an OD600 of 0.05. For the *B. longum* subspecies *infantis* and *bifidum* mix the individual precultures were first mixed in an equal amount based on OD600 and subsequently also inoculated with a start OD600 of 0.05. The medium was stirred at 360 rpm by magnet stirring bar. The pH was controlled at 6.2 by addition of 5M NaOH. All growth (precultures, main fermenters, agar plates) experiments were performed at 37°C. Growth was monitored in time by off-line OD600 measurement. Samples were diluted before measurement where appropriate.

The results are shown in Table 2.

**Table 2: Growth of Bifidobacterium strains, expressed as OD600, with or without urea and with or without CO₂.**

| | | | Fermentation time | | | | |
|---|---|---|---|---|---|---|---|
| Gas | Bifidobacteria | N source | 60 min | 180 min | 360 min | 660 min | 23 h |
| N₂ | urease positive | - | 0.094 | 0.127 | 0.160 | 0.226 | 0.305 |
| N₂ | urease positive | urea | 0.095 | 0.161 | 0.560 | 3.930 | 3.700 |
| N₂ | urease negative | - | 0.067 | 0.092 | 0.116 | 0.141 | 0.144 |
| N₂ | urease negative | urea | 0.051 | 0.061 | 0.072 | 0.054 | 0.046 |
| CO₂ | urease positive | - | 0.101 | 0.239 | 1.220 | 3.100 | 2.890 |
| CO₂ | urease positive | urea | 0.099 | 0.168 | 0.680 | 3.940 | 3.850 |
| CO₂ | urease negative | - | 0.070 | 0.188 | 0.870 | 1.830 | 2.710 |
| CO2 | urease negative | urea | 0.062 | 0.083 | 0.211 | 1.560 | 2.250 |

Urease positive Bifidobacteria hardly grow in the absence of both CO₂ and urea, but here growth can be restored in the absence of CO₂ by the presence of urea. This is indicative of urea serving both as a CO₂ and nitrogen source for the growth. In the presence of CO₂ and absence of urea on the other hand only an intermediate growth is observed, due to the restriction on nitrogen source. The highest growth was observed with urea, and the additional presence of CO₂ did not have an extra effect.

A second experiment was performed. Here the growth with different concentrations urea as nitrogen (and CO₂) source was compared to Tryptone (from Oxoid) as nitrogen source. In this case only *B*. *longum* ssp. *infantis* strains M-63, ATCC17930, DN_163_0041 and R-0033 were used and tryptone was removed from the basal medium. Where applicable tryptone or urea were added as nitrogen source to final concentrations of 3 g/l and 10, 50 or 100 mM, respectively. Instead of lactose as carbon source scGOS/lcFOS in a 9:1 wt/wt ratio was used in a final concentration of 20 g/l. As a source of scGOS VivinalGOS (FrieslandCampina) was used, and RaftininHP (Orafti) was used as IcFOS source.

The results are shown in Table 3.

**Table 3: Growth of Bifidobacterium strains (expressed as OD600) with or without urea and with or without CO₂.**

| | | | Fermentation time | | | | | |
|---|---|---|---|---|---|---|---|---|
| Gas | Bifidobacteria | N source | 15 min | 135 min | 270 min | 445 min | 625 min | 24 h |
| N₂ | urease positive | - | 0.083 | 0.112 | 0.140 | 0.130 | 0.160 | 0.140 |
| N₂ | urease positive | 10 mM urea | 0.084 | 0.137 | 0.390 | 1.280 | 3.900 | 3.720 |
| N₂ | urease positive | 50 mM urea | 0.082 | 0.140 | 0.450 | 1.260 | 2.970 | 2.850 |
| N₂ | urease positive | 100 mM urea | 0.079 | 0.144 | 0.430 | 1.130 | 2.800 | 2.520 |
| CO₂ | urease positive | - | 0.078 | 0.159 | 0.420 | 0.670 | 0.860 | 1.270 |
| CO₂ | urease positive | 50 mM urea | 0.079 | 0.161 | 0.670 | 2.610 | 6.500 | 6.5 |
| CO₂ | urease positive | tryptone 3 g/l | 0.080 | 0.168 | 0.810 | 2.300 | 2.810 | 3.210 |
| CO₂ | urease negative | 50 mM urea | 0.074 | 0.125 | 0.280 | 0.460 | 0.590 | 1.020 |

It can be deduced that urease negative Bifidobacteria again showed an intermediate growth in the presence of urea and CO₂.

Urease positive Bifidobacteria hardly grow in the absence of both CO₂ and urea, but in the absence of CO₂ and presence of urea growth is restored. Upon testing several concentrations, it seems that a concentration of 10 mM is already sufficient and 100 mM urea is less optimal. 10 mM urea corresponds to an amount of 60 mg urea per 100 ml. In the presence of CO₂ and absence of urea as nitrogen source on the other hand, an intermediate growth is observed, due to the restriction on nitrogen source. 10 mM urea and 3 g/l tryptone corresponds to about the same concentration of nitrogen and it can be deduced that urea is at least as good a nitrogen source as tryptone.

Controls without GOS/FOS or lactose did not show growth, due to the limited presence of a carbon-and energy source and this was the case for all bacterial strains (data not shown). In a control experiment the urease negative bacteria were able to grow very well in the presence of CO₂, GOS/FOS and tryptone as nitrogen source (data not shown), but very limited in the absence of tryptone.

The results show that the best growth is observed only in the presence of a combination of non-digestible oligosaccharides as carbon source and urea as both nitrogen and CO₂ source.

This is also indicative of a cross feeding effect, an effect going beyond the effect on the urease positive strains alone, wherein the presence of urease positive Bifidobacteria will beneficially affect the growth of other Bifidobacterium strains that are urease negative, by producing the growth factor CO₂ and by releasing ammonium as nitrogen source. This released CO₂ and ammonia can then be used by urease negative Bifidobacteria resulting in a more diverse and further improved microbiota. Therefore a symbiotic, improved or synergistic effect is to be expected from urea together with non-digestible oligosaccharides, in particular non-digestible oligosaccharides that stimulate the growth of urease positive Bifidobacteria.

### Example 4: Non-digestible oligosaccharides together with urea are superior in stimulating growth of Bifidobacteria

In this experiment the urease positive strain *Bifidobacterium longum* subspecies *infantis* ATCC 17930, was used.

The same growth media and experimental set up as in the second experiment described in example 3 was used, except that instead of scGOS/IcFOS, 2'-fucosyllactose (Jennewein Biotechnologie GmbH) was used as a carbon and energy source. As a nitrogen source tryptone was added (3.0 g/l), or no urea or 10 mM urea (final concentration), corresponding to 0, or 0.6 g/l urea.

The results of the growth experiment are shown in Table 4.

**Table 4: Growth of Bifidobacterium strain ATCC 17930, expressed as OD600, with or without urea and with or without CO₂.and with or without 2'FL as carbon and energy source (C source)**

| | | | Fermentation time | | | | |
|---|---|---|---|---|---|---|---|
| Gas | N source | C source | 30 min | 240 min | 360 min | 540 min | 24 h |
| N₂ | 3 g/l tryptone | + | 0.069 | 0.088 | 0.096 | 0.104 | 0.124 |
| N₂ | 3 g/l tryptone | - | 0.074 | 0.096 | 0.103 | 0.110 | 0.079 |
| N₂ | 10 mM urea | + | 0.079 | 0.154 | 0.350 | 0.600 | 1.320 |
| N₂ | 10 mM urea | - | 0.068 | 0.175 | 0.179 | 0.163 | 0.157 |
| CO₂ | 3 g/l tryptone | + | 0.069 | 0.305 | 0.940 | 2.000 | 3.290 |
| CO₂ | 3 g/l tryptone | - | 0.070 | 0.202 | 0.198 | 0.192 | 0.180 |
| CO2 | 10 mM urea | + | 0.072 | 0.298 | 0.880 | 2.070 | 3.060 |
| CO₂ | 10 mM urea | - | 0.071 | 0.203 | 0.191 | 0.191 | 0.187 |

Urease positive Bifidobacteria hardly grow in the absence of both CO₂ and urea, but again growth can be restored in the absence of CO₂ by the presence of 2'FL and urea. Controls without 2'FL did not show growth, due to the limited presence of a carbon- and energy source. These results are indicative for that the type of non-digestible oligosaccharides is not crucial for their effect on growth in the presence of urea, as long as the Bifibidobacteria can utilize them as a carbon and energy source.

### Example 5:

An infant formula comprising per 100 ml (to be reconstituted from 13.6 g powder with 90 ml water):
- 66 kcal
- 1.345 g total protein (based on total N * 6,25) whey protein/casein in 6/4 weight ratio and including 25 mg urea
- 6.9 g lactose
- 3.36 g fat (mixture of vegetable oil, fish oil, comprising LA, ALA, DHA and ARA)
- 0.8 g non-digestible oligosaccharides (scGOS/lcFOS in a 9:1 ratio)
- minerals, trace elements, vitamins and other micronutrients as known in the art and according to international guidelines for infant formula; additional NPN containing nutrients are 2.2 mg nucleotides, 1.6 mg carnitine, 12 mg choline and 5.3 mg taurine.

## Claims

1. A nutritional composition, which is an infant formula, a follow on formula or a young child formula, comprising digestible carbohydrates, protein, lipid, urea and non-digestible oligosaccharides, wherein the urea is present in an amount of
a. at least 1.13 wt%, preferably 1.13 to 5.6 wt%, based on total protein,
b. at least 0.11 wt%, preferably 0.11 to 0.55 wt%, based on dry weight of the composition, and/or
c. at least 15 mg, preferably 15 to 75 mg, per 100 ml, and non-digestible oligosaccharides are present in an amount of
d. at least 1.5 wt%, preferably 1.5 to 15 wt%, based on dry weight of the composition, and/or
e. at least 0.2 g, preferably 0.2 to 2 g, per 100 ml.

2. The nutritional composition according to claim 1, which is an infant formula.

3. The nutritional composition according to claim 1 or 2, wherein the urea is present in an amount of 15 to 75 mg per 100 ml.

4. The nutritional composition according to any one of the preceding claims, wherein the non-digestible oligosaccharides are selected from the group consisting of fructo-oligosaccharides, galacto-oligosaccharides, arabino-oligosaccharides, arabinogalacto-oligosaccharides, gluco-oligosaccharides, chito-oligosaccharides, glucomanno-oligosaccharides, galactomanno-oligosaccharides, mannan-oligosaccharides, fuco-oligosaccharides, sialyl-oligosaccharides and N acetylglucosamine oligosaccharides.

5. The nutritional composition according to any one of the preceding claims, wherein the non-digestible oligosaccharides are at least one, preferably two selected from the group consisting of galacto-oligosaccharides and fructo-oligosaccharides.

6. The nutritional composition according to any one of the preceding claims, wherein the non-digestible oligosaccharides comprise 2'-fucosyllactose.

7. The nutritional composition according to any one of the preceding claims comprising 2.1 g or less total protein per 100 kcal.

8. The nutritional composition according to any one of the preceding claims comprising at least 90 wt% cow's milk proteins based on total protein or comprising at least 90 wt% sheep milk proteins based on total protein or comprising at least 90 wt% goat milk proteins based on total protein.

9. The nutritional composition according to any one of the preceding claims further comprising at least one strain of a urease positive Bifidobacterium, preferably *B*. *longum* spp *infantis.*

10. The nutritional composition according to any one of the preceding claims further comprising at least one strain of a urease negative Bifidobacterium, preferably *B*. *breve.*

11. The nutritional composition according to any one of claims 1-10, for use in preventing or treating dysbiosis of the intestinal microbiota in infants or young children.

12. The nutritional composition according to any one of claims 1-10, for use in preventing and/or treating disorders associated with a compromised intestinal microbiota in infants or young children.

13. The nutritional composition for the use according to claim 12, wherein said use is for preventing and/or treating a disorder selected from the group consisting of diarrhea, constipation, intestinal inflammation, intestinal infection and allergy.

## Patentansprüche

1. Ernährungszusammensetzung, die eine Säuglingsnahrung, eine Folgenahrung oder eine Kleinkindnahrung ist, die verdauliche Kohlenhydrate, Protein, Lipid, Harnstoff und unverdauliche Oligosaccharide umfasst, wobei der Harnstoff in einer folgenden Menge vorhanden ist
a. mindestens 1,13 Gew.-%, vorzugsweise 1,13 bis 5,6 Gew.-%, bezogen auf das Gesamtprotein,
b. mindestens 0,11 Gew.-%, vorzugsweise 0,11 bis 0,55 Gew.-%, bezogen auf das Trockengewicht der Zusammensetzung, und/oder
c. mindestens 15 mg, vorzugsweise 15 bis 75 mg, pro 100 ml, und unverdauliche Oligosaccharide in einer folgenden Menge vorhanden sind
d. mindestens 1,5 Gew.-%, vorzugsweise 1,5 bis 15 Gew.-%, bezogen auf das Trockengewicht der Zusammensetzung, und/oder
e. mindestens 0,2 g, vorzugsweise 0,2 bis 2 g, pro 100 ml.

2. Ernährungszusammensetzung nach Anspruch 1, die eine Säuglingsnahrung ist.

3. Ernährungszusammensetzung nach Anspruch 1 oder 2, wobei der Harnstoff in einer Menge von 15 bis 75 mg pro 100 ml vorhanden ist.

4. Ernährungszusammensetzung nach einem der vorhergehenden Ansprüche, wobei die unverdaulichen Oligosaccharide aus der Gruppe ausgewählt sind, die aus Folgendem besteht: Fructo-Oligosacchariden, Galacto-Oligosacchariden, Arabino-Oligosacchariden, Arabinogalacto-Oligosacchariden, Gluco-Oligosacchariden, Chito-Oligosacchariden, Glucomanno-Oligosacchariden, Galactomanno-Oligosacchariden, Mannan-Oligosacchariden, Fuco-Oligosacchariden, Sialyl-Oligosacchariden und N-Acetylglucosamin-Oligosacchariden.

5. Ernährungszusammensetzung nach einem der vorhergehenden Ansprüche, wobei die unverdaulichen Oligosaccharide mindestens eines, vorzugsweise zwei sind, die aus der Gruppe ausgewählt sind, die aus Galacto-Oligosacchariden und Fructo-Oligosacchariden besteht.

6. Ernährungszusammensetzung nach einem der vorhergehenden Ansprüche, wobei die unverdaulichen Oligosaccharide 2'-Fucosyllactose umfassen.

7. Ernährungszusammensetzung nach einem der vorhergehenden Ansprüche, die 2,1 g oder weniger Gesamtprotein pro 100 kcal umfasst.

8. Ernährungszusammensetzung nach einem der vorhergehenden Ansprüche, die mindestens 90 Gew.-% Kuhmilchproteine umfasst, bezogen auf das Gesamtprotein, oder die mindestens 90 Gew.-% Schafmilchproteine umfasst, bezogen auf das Gesamtprotein, oder die mindestens 90 Gew.-% Ziegenmilchproteine umfasst, bezogen auf das Gesamtprotein.

9. Ernährungszusammensetzung nach einem der vorhergehenden Ansprüche, die ferner mindestens einen Stamm eines Urease-positiven Bifidobakteriums umfasst, vorzugsweise *B*. *longum* spp *infantis.*

10. Ernährungszusammensetzung nach einem der vorhergehenden Ansprüche, die ferner mindestens einen Stamm eines Urease-negativen Bifidobakteriums umfasst, vorzugsweise *B*. *breve.*

11. Ernährungszusammensetzung nach einem der Ansprüche 1-10 zur Verwendung bei der Vorbeugung oder Behandlung von Dysbiose der Darmmikrobiota bei Säuglingen oder Kleinkindern.

12. Ernährungszusammensetzung nach einem der Ansprüche 1-10 zur Verwendung bei der Vorbeugung und/oder Behandlung von Erkrankungen, die mit einer beeinträchtigten Darmmikrobiota bei Säuglingen oder Kleinkindern assoziiert sind.

13. Ernährungszusammensetzung zur Verwendung nach Anspruch 12, wobei die besagte Verwendung zur Vorbeugung und/oder Behandlung einer Erkrankung dient, die aus der Gruppe ausgewählt ist, die aus Diarrhöe, Obstipation, Darmentzündung, Darminfektion und Allergie besteht.

## Revendications

1. Composition nutritionnelle, qui est une préparation pour nourrissons, une préparation de suite ou une préparation pour jeunes enfants, comprenant des glucides digestibles, des protéines, des lipides, de l'urée et des oligosaccharides non digestibles,
où l'urée est présente en une quantité de
a. au moins 1,13 % en poids, de préférence 1,13 à 5,6 % en poids, sur la base des protéines totales,
b. au moins 0,11 % en poids, de préférence 0,11 à 0,55 % en poids, sur la base du poids sec de la composition, et/ou
c. au moins 15 mg, de préférence 15 à 75 mg, par 100 ml,
et les oligosaccharides non digestibles sont présents en une quantité de
d. au moins 1,5 % en poids, de préférence 1,5 à 15 % en poids, sur la base du poids sec de la composition, et/ou
e. au moins 0,2 g, de préférence 0,2 à 2 g, par 100 ml.

2. Composition nutritionnelle selon la revendication 1, qui est une formule infantile.

3. Composition nutritionnelle selon la revendication 1 ou 2, où l'urée est présente à raison de 15 à 75 mg par 100 ml.

4. Composition nutritionnelle selon l'une quelconque des revendications précédentes, où les oligosaccharides non digestibles sont choisis dans le groupe consistant en fructo-oligosaccharides, galacto-oligosaccharides, arabino-oligosaccharides, arabinogalacto-oligosaccharides, gluco-oligosaccharides, chito-oligosaccharides, glucomanno-oligosaccharides, galactomanno-oligosaccharides, mannan-oligosaccharides, fuco-oligosaccharides, sialyl-oligosaccharides et oligosaccharides de N acetylglucosamine.

5. Composition nutritionnelle selon l'une quelconque des revendications précédentes, où les oligosaccharides non digestibles sont au moins un, de préférence deux choisis dans le groupe consistant en galacto-oligosaccharides et fructo-oligosaccharides.

6. Composition nutritionnelle selon l'une quelconque des revendications précédentes, où les oligosaccharides non digestibles comprennent du 2'-fucosyllactose.

7. Composition nutritionnelle selon l'une quelconque des revendications précédentes comprenant 2,1 g ou moins de protéines totales par 100 kcal.

8. Composition nutritionnelle selon l'une quelconque des revendications précédentes comprenant au moins 90 % en poids de protéines de lait de vache sur la base des protéines totales ou comprenant au moins 90 % en poids de protéines de lait de brebis sur la base des protéines totales ou comprenant au moins 90 % en poids de protéines de lait de chèvre sur la base des protéines totales.

9. Composition nutritionnelle selon l'une quelconque des revendications précédentes comprenant en outre au moins une souche de Bifidobacterium uréase positive, de préférence *B*. *longum* spp *infantis.*

10. Composition nutritionnelle selon l'une quelconque des revendications précédentes comprenant en outre au moins une souche de Bifidobacterium uréase négative, de préférence B. *breve.*

11. Composition nutritionnelle selon l'une quelconque des revendications 1 à 10, pour utilisation dans la prévention ou le traitement de la dysbiose du microbiote intestinal chez les nourrissons ou les jeunes enfants.

12. Composition nutritionnelle selon l'une quelconque des revendications 1 à 10, pour utilisation dans la prévention et/ou le traitement de troubles associés à un microbiote intestinal affaibli chez les nourrissons ou les jeunes enfants.

13. Composition nutritionnelle pour utilisation selon la revendication 12, où ladite utilisation est pour prévenir et/ou traiter un trouble choisi dans le groupe consistant en diarrhée, constipation, inflammation intestinale, infection intestinale et allergie.
